# EUROPEAN PATENT APPLICATION

(11) **EP 1 417 961 A1**
(43) Date of publication of application: **12.05.2004**
(21) Application number: 02024891.0
(22) Date of filing: 08.11.2002
(51) Int. Cl.: A61K 31/165

(54) **New pharmaceutical compositions containing a combination of ambroxol or bromhexine and isopropamide iodide**

(71) Applicant: Boehringer Ingelheim International GmbH, 55216 Ingelheim am Rhein (DE)
(72) Inventor: Umehara, Norimitsu, Tokorozawa-shi, Saitama, 359-0025 (JP); Miyadai, Nobuo, Narita, Chiba, 286-0048 (JP); Okada, Minoru, Inzai, Chiba, 270-1323 (JP)

(57) **Abstract**

The present invention relates to novel pharmaceutical compositions comprising as pharmacologically active compounds a combination of an expectorant-effective amount of bromhexine or ambroxol or a pharmacologically acceptable salt thereof and a parasympatholytic (anticholinergic)-effective amount of isopropamide iodide.

Qualitative and quantitative formula of this invention includes pharmaceutically acceptable carriers or excipients.

The formulation further comprises suitable pharmaceutically acceptable carriers or excipients.

In addition, this invention is related to the usage of these compounds for the various symptoms caused by the common cold.

Especially, the composition of the present invention is effective in the treatment of sputum and/or runny nose among the various symptoms caused by the common cold.

## Description

The present invention relates to novel pharmaceutical compositions comprising as pharmacologically active compounds a combination of an expectorant-effective amount of bromhexine or ambroxol or a pharmacologically acceptable salt thereof and a parasympatholytic (anticholinergic)-effective amount of isopropamide iodide. The formulation further comprises suitable pharmaceutically acceptable carriers or excipients. Additionally, the present composition may contain other pharmaceutically active compounds furthermore.
Another aspect of the present invention relates to methods of using these compositions in the treatment of symptoms of the common cold.
In particular the inventive composition is useful in the treatment of expectoration and/or runny nose in the many symptoms of the common cold.

### Background of the invention

Common cold is a condition that various reactions occurred when stimulated by the microbes or chilliness to the respiratory organ and it is an acute inflammation in respiratory organ, which is an airway from mouth, nose through lungs. More precisely, it is called "common cold syndrome"

Nose inflammation caused by the common cold is called acute rhinitis and brings about symptoms such as sneeze, runny or stuffy nose. In the acute adenoiditis which is an inflammation of the throat, congestion of the mucous membrane of the throat, swelling and pain and other symptoms occur. When the infection affects the bottom of the respiratory organ, hoarse voice and sometimes dyspnea occur and once it reached bronchus, bronchial and lungs, onset of coughs and sputum begins. In addition to the above-mentioned symptoms in the respiratory organ, headache, fever, low back pain, weariness and anorexia of the whole body appears. Furthermore, gastro symptoms including abdominal pain and diarrhea sometimes occur.

Although coldness and allergic reaction are partly attributed to the onset of common cold, many of it is caused by viral infection. The kind of virus causes the common cold is said to be more than 200. However, very few drugs are effective for the virus at present. For this reason, symptomatic treatment to control symptoms of common cold such as runny nose, stuffy nose, sneeze, sputum, throat pain, fever and muscle pain is the main pharmaceutical therapy.

However, drug effective for all symptoms has not been developed yet. Therefore, combination drug manufactured for each symptom is used for the treatment.

Therefore it is an objective of the present invention to offer extremely effective pharmaceutical compositions that improve sputum and/or runny nose.

Another objective of the present invention is to offer a medicine for a cold using extremely effective pharmaceutical compositions that improve effectively sputum and/or runny nose among the symptoms of the common cold.

### Description of the invention

Thus it has surprisingly been found that a pharmaceutical composition according to the present invention comprising as pharmacologically active compounds a combination of an expectorant-effective amount of bromhexine or ambroxol or a pharmacologically acceptable salt thereof and a parasympatholytic (anticholinergic)-effective amount of isopropamide iodide, is suitable for treating the symptoms of common cold.

The present invention relates to pharmaceutical compositions comprising as pharmacologically active compounds a combination of an expectorant-effective amount of bromhexine or ambroxol, preferably ambroxol, or a pharmacologically acceptable salt thereof and a parasympatholytic (anticholinergic)-effective amount of isopropamide iodide.

Ambroxol, to be used for pharmaceutical compositions of the present invention, chemical name: trans-4- [2-amino-3, 5-dibromobenzyl] amino] cyclohexanol, is an expectorant classified into mucosal lubricant drug, which, by the increase in production of pulmonary surfactant, has the effect to lubricate membrane of the airway, where sputum is coughing up. Ambroxol is a metabolite of bromhexine.

In the present invention, preferably ambroxol hydrochloride is used. However, other acid addition salt including hydrobromate, oxalate, nitrate, sulphonate, fumarate, maleate, sulfate phosphate, and the like or freebase can also be used.

In the present invention, bromhexine may be used instead of part or all of the ambroxol.

Bromhexine, to be used for pharmaceutical compositions of the present invention, chemical name: 2-amino-3, 5-dibromo-N-cyclohexyl-N-methylbenzylamine, is an expectorant classified into airway secretagogue, which has the effect to increase the airway secretion.

In the present invention, preferably bromhexine hydrochloride is used. However, other acid addition salt including hydrobromate, oxalate, nitrate, sulphonate, fumarate, maleate, sulfate phosphate, and the like or freebase can also be used.

In context of the present invention bromhexine or its pharmacologically acceptable salt may be blended with the isopropamide iodide in an amount of 1.2 to 32 mg as daily dosage for adults, 8 to 16 mg is more preferable, and 12 mg is most preferable.

In context of the present invention ambroxol or its pharmacologically acceptable salt may be blended with the isopropamide iodide in an amount of 5 to 90 mg as daily dosage for adults, 10 to 60 mg is more preferable, and 22.5 to 45 mg is most preferable.

Isopropamide iodide, the chemical name: 3-Carbamoyl-3, 3-diphenylpropyl) diisopropylmethylammonium iodide, to be used in the pharmaceutical compositions in the present invention is anticholinergic drug which block the parasympathetic nerve and suppress the excessive secretion of membrane and moderate the runny nose.

The amount of isopropamide iodide is 1 to 25 mg as daily dosage for adults, 2 to 10 mg is more preferable, and 3 to 6 mg is most preferable.

In this present invention, mixture ratio of isopropamide iodide to ambroxol, preferably in the form of its pharmacologically acceptable salt, e.g. ambroxol hydrochloride, is commonly in the range of 0.01 to 5 weight part. The range is preferably, 0.04 to 1 weight part and more preferably, 0.1 to 0.3 weight part.

The pharmaceutical compositions in the present invention can be administered orally in single or multiple doses. In addition, dosage of ambroxol or its pharmacologically acceptable salt and isopropamide iodide can be adjusted according to age, weight symptom, and the like.

In addition, in the pharmaceutical compositions in this invention, one or more than two substance(s) selected from the group comprising as pharmacologically active substance antipyretic analgesics, antihistamine, antitussive, stimulant drug, vitamins, crude drug, antacid, mucosa protective as covering materials, antiphlogistic, quenching enzyme and expectorant.

Each amount of these pharmacologically active substances is decided according to a well-known combination standard in consideration of other kinds and quantity of pharmacologically active substances that are used together.

Examples of antipyretic analgesics are ibuprofen, acetaminophen, ethenzamide, aspirin, aluminum aspirin, isopropylantipyrine, sasapyrine, salicylamide, sodium salicylate, lactyl phenetidine, and the like. They can be used either singly or in their two or more combinations. The amount of antipyretic analgesics is commonly 10 to 5000 mg as daily dosage for adults and preferably, it is 225 to 3000 mg.

Examples of antihistamic agent are isothipendyl hydrochloride, diphenylpyraline hydrochloride, diphenhydramine hydrochloride, difeterol hydrochloride, triprolidine hydrochloride, tripelennamine hydrochloride, thonzylamine hydrochloride, fenethazine hydrochloride, methdilazine hydrochloride, diphenhydramine salicylate, carbinoxamine diphenyl disulphonate, alimemazine tartrate, diphenhydramine tannate, diphenylpyraline teoclate, mebhydrolin napadisylate, promethazine methylene two salicylates, carbinoxamine maleate, chlorpheniramine maleate, difeterol phosphate, mequitazine., promethazine, cyproheptazine hydrochloride, iproheptine hydrochloride, clemastine fumarate and epinastine hydrochloride, and the like. They can be use either singly or in their two or more combinations. The amount of antihistamic agent is commonly 1 to 300 mg as daily dosage for adults and preferably, it is 1.5 to 150 mg.

Examples of antitussive are alloclamide hydrochloride, hydrochloric acid chloperastine, tipepidine citrate, sodium dibunate, dextromethorphan hydrobromide, dextromethorphan - phenol cover microcosmic salt, tipepidine hibenzate, cloperastine fendizoate, codeine phosphate, dihydrocodeine phosphate pentoxyverine citrate, noscapine hydrochloride, noscapine, dl- methylephedrine hydrochloride, dl- methylephedrine saccharin salt, carbetapentane citrate, dimemorfan phosphate, benproperine phosphate, isoaminile citrate, oxeladin citrate, oxeladin tannate, eprazinone hydrochloride, clobutinol hydrochloride, clofedanol hydrochloride, fominoben hydrochloride, I-methylephedrine hydrochloride, trimetoquinol hydrochloride, pseudoephedrine, phenylpropanolamine hydrochloride.methoxyphenamine hydrochloride, and the like. They can be use either singly or in their two or more combinations. The amount of the antitussive is commonly 2 to 900 mg as daily dosage for adults and preferably, it is 12 to 90 mg.

Examples of stimulant drugs are di-methylephedrine hydrochloride, dl- methylephedrine saccharin salt, caffeine and sodium benzoate, caffeine, anhydrous caffeine, ephedrine hydrochloride, pseudoephedrine, phenylpropanolamine hydrochloride, phenylephrine, 1- methylephedrine hydrochloride, methoxyphenamine hydrochloride, dl- epinephrine hydrochloride, dl- isoproterenol hydrochloride, isoproterenol sulfate, orciprenaline sulfate, terbutaline sulfate, salbutamol sulfate, trimetoquinol hydrochloride, hexoprenaline sulfate, clorprenaline hydrochloride, tulobuterol hydrochloride, procaterol hydrochloride, pirbuterol hydrochloride, fenoterol hydrobromide, formoterol fumarate, clenbuterol hydrochloride, mabuterol hydrochloride, hydrochloric acid ethylcysteine, methyl cysteine hydrochloride, and the like. They can be use either singly or in their two or more combinations. The amount of the stimulant is commonly 1 to 900 mg as daily dosage for adults and preferably, it is 5 to 600 mg.

Examples of vitamins are vitamin B₁ and the derivative and that salts such as octotiamine, prosultiamine, fursultiamine, hydrochloric acid fursultiamine, bisbentiamine, benfotiamine, dicethiamine hydrochloride, cycotiamine, cocarboxylase, thiamin disulfide, thiamine hydrochloride, thiamin mononitrate, bisthiamine nitrate, thiamine di- cetyl sulfate salt, bisibuthiamine, and the like, vitamin B₂ and the derivative and that salts such as riboflavin, riboflavin tetrabutyrate, riboflavin sodium phosphate, flavin adenine dinucleotide sodium, and the like, vitamin C and the derivative and that salts such as ascorbic acid, sodium ascorbate, calcium ascorbate, and the like, hesperidin and the derivative and that salt, vitamin F, vitamin A such as retinol acetate, retinol palmitate and the derivative and those salt, vitamin E and the derivative and that salt such as the tocopherol, tocopherol succinate, tocopherol calcium succinate, tocopherol acetate, and the like. They can be use either singly or in their two or more combinations. The amount of the vitamins is commonly 0.1 to 2000 mg as defined daily dosage for adult and preferably, it is 1 to 500 mg.

Examples of crude drug are crude drug powder and/or the extract such as Ephedra Herb, Nandina Fruit, Cherry Bark, Polygala Root, Glycyrrhiza, Platycodon Root, Plantago Seed, Plantago Herb, Lycoris Radiata Herb, Senega, Fritillaria, Fennel, Phellodendron Bark, Coptis Rhizome, Zedoary, Chamomile, Cinnamon Bark, Gentian, Oriental Bezoar, Bear Bile, Glehnia Root, Ginger, Atractylodes Lancea Rhizome, Clove, Citrus Unshiu Peel, Atractylodes Rhizome, Diryu (Earthworm), Panax Rhizome, Ginseng, Scutellaria Root, Pueraria Root, Apricot Kernel, Cyperus Rhizome, Nonglutinous Rice, Magnolia Bark, Schisandra Fruit, Bupleurum Root, Asiasarum Root, Peony Root, Perilla Herb, Jujube, Ophiopogon Tuber, Pinellia tuber, Poria Sclerotium, Kakkon-to, Keishi-to, Kososan, Saiko-keishi-to, Sho-saiko-to, Sho-seiryu-to, Bakumondo-to, Hange-kobokuto, Mao-to, Schizonepeta Spike, Forsythia Fruit, Polygala Root, Magnolia, Peach Kernel, Aconite Root, and the like. They can be use either singly or in their two or more combinations. The amount of the crude drug is commonly 0.01 to 300 g in extract (converted into raw crude drug) and/or 0.0001 to 60g in powder drug as daily dosage for adults and preferably, it is 0.05 to 30 g in extract (converted into raw crude drug) and/or 0.002 to 6 g in powder.

Examples of antacid and mucosa protective are aminoacetate, magnesium oxide, magnesium carbonate, magnesium silicate, synthetic aluminum silicate, synthetic hydrotalcite, dihydro aluminum-aminoacetate salt, aluminum hydroxide gel, dried aluminum hydroxide gel, aluminum hydroxide-magnesium carbonate mixing dried gel, aluminum hydroxide-sodium bicarbonate co-precipitate, aluminum hydroxide-calcium carbonate-magnesium carbonate co-precipitate, magnesium hydroxide-potassium aluminum sulfate co-precipitate, magnesium aluminometasilicate and the like. They can be use either singly or in their two or more combinations. The amount of antacid and mucosa protective is commonly 10 to 8000 mg as daily dosage for adults and preferably, it is 100 to 4000 mg.

Examples of antiphlogistic and quenching enzyme are bromelain, pronase, serrapeptase, semi- alkali proteinase, streptokinase, streptodornase, lysozyme chloride, tranexamic acid, and the like. They can be use either singly or in their two or more combinations. The amount of anti-inflammatory enzyme preparations is commonly 4 to 2000 mg as daily dosage for adults and preferably, it is 15 to 720 mg.

Expectorants excluding ambroxol include Potassium guaiacolsulfonate, guaifenesin, potassium iodide, foeniculated ammonia spirit, sodium hydrogencarbonate, bromhexine hydrochloride, fudosteine, carbocysteine, methyl cysteine hydrochloride, acetylcysteine, ethylcysteine hydrochloride, eprazinone hydrochloride, aminophylline, theophylline, diprophylline, proxyphylline, ammonium chloride, cresol potassium sulphonate, I- menthol, trimetoquinol hydrochloride, phenylpropanolamine hydrochloride, methoxyphenamine hydrochloride, and the like. They can be use either singly or in their two or more combinations. The amount of expectorant excluding ambroxol is commonly 1 to 3000 mg as daily dosage for adults and preferably, it is 6 to 900 mg.

Pharmaceutical compositions of this invention are used as solid, semi-solid and liquid preparations of oral administration such as tablets, granule, subtle granules, powder, capsule, couplet, soft capsule, pill, suspension, emulsion, liquid, syrup, dry syrup, and the like. Moreover, these preparations may be manufactured after make them into micro particles such as microcapsule, nanocapsule, microsphere and nano sphere.

These preparations can be manufactured in usual manner, adding preparation additive to pharmacologically active substance if necessary and the manufacture method is not limited.

As an additive of the preparations to the pharmaceutical compositions of this invention, the following can be used and there were no limitations: stabilizer, surfactant, plasticizer, lubricant, solubilizer, reducing agent, buffer agent, sweetening agent, base, adsorbent, corrigent, binder, suspension, suspending agent, antioxidant, polish, coating, wetting agent, wet modifier, filler, antifoaming agent, refrigerative agent, coloring matter, flavoring agent, perfume, sugar coating agent, isotonizing agent, softener, emulsifying agent, foaming agent, pH modifier, diluent, excipient, dispersing agent, disintegrator, fragrance, desiccant, antiseptics, preservative, solubilizing agent, solubilizer, solvent, superplasticizer, antistatic agent, extender, moisturizing agent, and the like.

Examples of additives are lactose, sucrose, glucose, mannitol, sorbitol, potato starch, corn starch, wheat starch, calcium carbonate, calcium sulfate, sodium hydrogencarbonate, sodium chloride.microcrystalline cellulose, methyl cellulose, ethyl cellulose, hydroxypropyl methylcellulose, hydroxypropyl cellulose, carboxymethylcellulose, sodium carboxymethylcellulose, carboxy methylcellulose calcium, polyvinyl alcohol, magnesium stearate, talc, hydrogenation vegetable oil, macrogol, silicone oil, agar, calcium carbonate, sodium hydrogencarbonate, sodium alginate, shellac, glycerin, aromatic essential oil, water-soluble food dye, rake pigment, benzoic acid, sodium benzoate, para oxybenzoic acid, ester, cationic soap, dehydroacetic acid, boric acid, chlorobutanol, benzyl alcohol, polysorbate 80, fatty acid ester of glycerin, white beeswax, medium-chain triglyceride, ascorbic acid, tocopherol, sodium thiosulfate, sodium edetate, and the like. Additives that can be used for the pharmaceutical compositions of this invention are not restricted to those mentioned above, if they are available in the manufacturing pharmacy.

For example, when pharmaceutical compositions of this invention are manufactured as tablets, granule, fine granule, powder, capsules, couplet, pill, dry syrup, in case granulated powders is need to be adjusted, they are manufactured by the generally used method including wet granulation method such as spray granulation, agitate granulation, flow granulation, roll flow granulation, roll granulation and dry granulation such as compaction granulation. In addition, powders and granulated powders which contains pharmacologically active substance are mixed and divided into small sachet and can be filled.

When manufacture as a capsule, filling powder medicine, granulated powders, small tables and others in the capsule by using capsule-filling machine.

Tablets and couplets are manufactured by mixing powder of active constituent, powder agent, fine grain agent, granulated powder or pill and additive of the preparation and put to compression molding.

Coated preparations including sugarcoated tablets, couplets, film-coated tablets and coated granulated powder are manufactured in the usual manner such as pan coating, flow coating and rolling coating methods and combination of these.
Internal medicine such as syrup, elixir, limonade, extract, drinkable preparation and soft and hard capsule agents filling liquid or semi-solid substance are normally manufactured by mixing, dissolving and suspending each pharmaceutically active agents and part of preparation additive such as resolvent like distilled water, adding preparation additive including remaining resolvent and adjusting the volume of liquid. pH adjustment can be conducted using acid or alkali in necessary. Furthermore, when fat-soluble ingredient is included, it may be solubilized, emulsificated and slurred by using preparation additive such as detergent, solublizing agent, emulsifier and suspending agents. If necessary at preparation, warming, cooling, nitrogen displacement, filtering and sterilization can be performed.
Moreover, functions can be added using preparation additive: improvement in stabilization, slow release, continuance, quickly distinglation, quickly dissolution and dissolution of medicinal properties, concealment of taste, improvement in usage. Adding these functions can be done by the usual manner. For example: dispensing pharmaceutically active substance in a separate granule, making multi-layer granules, multi-layer tablets or dry coated tablet, tablets by separating granules, microcapsules, coating preparations such as sugarcoated tablets, film coating tablets, coating granule, foaming pharmaceutical preparation, chewable preparation, dissolving preparation in the mouth, matrix preparation, together comminution, making solid solution, adding sweetening agent, refrigerant, antioxidant or stabilizing agent, adjust to certain pH, viscosity, osmotic pressure, salt concentration. These methods can be combined.

Compositions of this invention are detailed below showing examples. However, pharmaceutical compounds of this invention are not restricted to the following examples.

### Example 1

### Tablet

Tablet was manufactured by evenly mixing the following ingredients and that mixed powder was molded to have 120 mg per tablet by direct compression.

| | |
|---|---|
| Ambroxol hydrochloride | 135 g |
| Isopropamide iodide | 18 g |
| Lactose | 459 g |
| Crystalline cellulose | 450 g |
| Light anhydrous silicic acid | 8 g |
| Talc | 5 g |
| Magnesium stearate | 5 g |

### Example 2

### Powder medicine

Powder was manufactured by evenly mixing the following ingredients and that mixed powder was molded to have 600mg per sachet.

| | |
|---|---|
| Ambroxol hydrochloride | 45 g |
| Isopropamide iodide | 6 g |
| Acetaminophen | 900 g |
| Corn starch | 289 g |
| Lactose | 540 g |
| Magnesium stearate | 20 g |

### Example 3

### Syrup

Total of 240 ml syrup was manufactured by dissolving the following ingredients in distilled water.

| | |
|---|---|
| Ambroxol hydrochloride | 0.15 g |
| Isopropamide iodide | 0.02 g |
| Acetaminophen | 3.00 g |
| Dihydrocodeine phosphate | 0.08 g |
| di-Methylephedrine hydrochloride | 0.20 g |
| Chlorpheniramine maleate | 0.03 g |
| Absolute caffeine | 0.25 g |
| Trehalose | 120.00 g |
| Citric acid | 0.10 g |
| Sodium citrate | 0.10 g |
| Caramel | 0.10 g |
| Aroma chemical | 0.50 g |

### Example 4

### Sugarcoated tablet

Tablet powder was manufactured with the following ingredients in the usual manner and molded to have 270 mg per tablet.

| | |
|---|---|
| Ambroxol hydrochloride | 90 g |
| Isopropamide iodide | 12 g |
| Ibuprofen | 900 g |
| Dihydrocodeine phosphate | 48 g |
| dl-Methylephedrine hydrochloride | 120 g |
| Chlorpheniramine maleate | 15 g |
| Anhydrous caffeine | 150 g |
| Thiamine nitrate | 48 g |
| Ascorbic acid | 600 g |
| Corn starch | 1257 g |
| Lactose | 936 g |
| Crystallized cellulose | 360 g |
| Hydroxypropylcellulose | 180 g |
| Light anhydrous Silicic acid | 90 g |
| Talc | 36 g |
| Magnesium stearate | 18 g |

This tablet was coated in the coating pan, using coating liquid (ethyl alcohol: distilled water = 1: 1) containing 5% by weight hydroxypropylcellulose until the weight per tablet increased by 10 mg. Then, solution containing 2% by weight titanium oxide, 3% by weight calcium carbonate, 1 % weight by gum Arabic powder and 60% by weight sucrose was used to coat the tablets until the weight per tablet increased by 180 mg. Afterwards, solution containing sucrose of 60% by weight was used for coating until the weight of one tablet increased by 10 mg.

### Example 5

### Granule agent

Granule was manufactured by using the following ingredients in the usual manner and it was packed in cartridge to have 1300 mg to be a granule agent.

| | |
|---|---|
| Ambroxol hydrochloride | 90 g |
| Isopropamide iodide | 12 g |
| Ibuprofen | 900 g |
| Dihydrocodeine phosphate | 48 g |
| Methylephedrine hydrochloride | 120 g |
| Chlorpheniramine maleate | 15 g |
| Anhydrous caffeine | 150 g |
| Thiamine nitrate | 48 g |
| Ascorbic acid | 600 g |
| Corn starch | 293 g |
| D-mannitol | 5240 g |
| Tartaric acid | 200 g |
| Aspartame | 40 g |
| Acesulfame potassium | 40 g |
| Perfume | 4 g |

### Example 6

### Tablet

Tablet was manufactured by evenly mixing the following ingredients and that mixed powder was molded to have 300 mg per tablet by direct compression.

| | |
|---|---|
| Ambroxol hydrochloride | 45 g |
| Isopropamide iodide | 6 g |
| Ibuprofen | 450 g |
| Dihydrocodeine phosphate | 24 g |
| Mequitazine | 6 g |
| Pseudoephedrine hydrochloride | 60 g |
| Theophylline | 150 g |
| Lysozyme chloride | 90 g |
| Anhydrous caffeine | 75 g |
| Fursultiamine | 24 g |
| Riboflavin | 12 g |
| Lactose | 443 g |
| Crystalline cellulose | 390 g |
| Magnesium stearate | 15 g |
| Talc | 10 g |

### Example 7

### Tablet

Tablet was manufactured by evenly mixing the following ingredients and that mixed powder was molded to have 240 mg per tablet by direct compression.

| | |
|---|---|
| Ambroxol hydrochloride | 45 g |
| Isopropamide iodide | 6 g |
| Acetaminophen | 900 g |
| Dihydrocodeine phosphate | 24 g |
| dl-Methylephedrine hydrochloride | 60 g |
| Pseudoephedrine hydrochloride | 60 g |
| Epinastine hydrochloride | 10 g |
| Serrapeptase | 15 g |
| Anhydrous caffeine | 75 g |
| Benfotiamine | 24 g |
| Riboflavin | 12 g |
| Lactose | 464 g |
| Microcrystalline cellulose | 430 g |
| Magnesium stearate | 20 g |
| Talc | 15 g |

## Claims

1. Pharmaceutical composition comprising as pharmacologically active compounds a combination of bromhexine or ambroxol or a pharmacologically acceptable salt thereof and isopropamide iodide.

2. Pharmaceutical composition according to claim 1 comprising as pharmacologically active compounds a combination of ambroxol or a pharmacologically acceptable salt thereof and isopropamide iodide.

3. Pharmaceutical composition according to claim 2 comprising Ambroxol Hydrochloride.

4. Pharmaceutical composition according to one of claims 1 to 3 **characterized in that** the composition comprises 5 to 90 mg ambroxol hydrochloride or a pharmacologically acceptable salt thereof as daily dosage for adults.

5. Pharmaceutical composition according to one of claims 1 to 4 **characterized in that** the composition comprises 1 to 25 mg isopropamide iodide as daily dosage for adults.

6. Pharmaceutical composition according to one of claims 1 to 5 **characterized in that** the mixture ratio of isopropamide iodide to ambroxol is in the range of 0.01 to 5 weight part.

7. Pharmaceutical composition according to one of claims 1 to 6 **characterized in that** it comprises one or more pharmacologically active compounds selected from the group consisting of antipyretics, analgesics, antihistamines, antitussives, stimulant drugs, vitamins, crude drugs, antacids and mucosa protectives, antiphlogistics, quenching enzymes and expectorants.

8. Use of pharmaceutical composition according to one of claims 1 to 7 for the preparation of a medicament for treating common cold.

9. Use of pharmaceutical composition according to one of claims 1 to 7 for the preparation of a medicament for treating sputum and/or runny nose.

10. Solid, semi-solid or liquid preparation of pharmaceutical composition according to one of claims 1 to 7.
